# EUROPÄISCHE PATENTANMELDUNG

(11) **EP 2 108 371 A1**
(43) Veröffentlichungstag der Anmeldung: **14.10.2009**
(21) Anmeldenummer: 08006956.0
(22) Anmeldetag: 08.04.2008
(51) Int. Cl.: A61K 31/575, A61K 45/06, A61P 15/12, A61P 15/18, C07J 9/00

(54) **Estra-1,3,5(10)-trien-Derivate zur Kontrazeption**

(71) Anmelder: Grünenthal GmbH, 52078 Aachen (DE)
(72) Erfinder: Pfaff, Tamara, Dr., D-40219 Düsseldorf (DE); Otten, Thomas, Dr., D-52159 Roetgen (DE)
(74) Vertreter: Bülle, Jan

(57) **Zusammenfassung**

Die Erfindung betrifft pharmazeutische Zusammensetzungen und Darreichungsformen, welche Verbindungen der allgemeinen Formel (I) 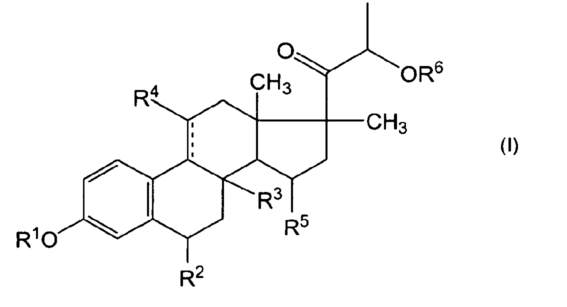 enthalten, und Verfahren zur Kontrazeption durch Verabreichung dieser Darreichungsformen. Ferner betrifft die Erfindung Verbindungen der allgemeinen Formel (I) als Medikament und die Verwendung dieser Verbindungen zur Herstellung von Arzneimitteln zur Hormonersatz-Therapie.

## Beschreibung

Die Erfindung betrifft pharmazeutische Zusammensetzungen und Darreichungsformen, welche Verbindungen der allgemeinen Formel (I) 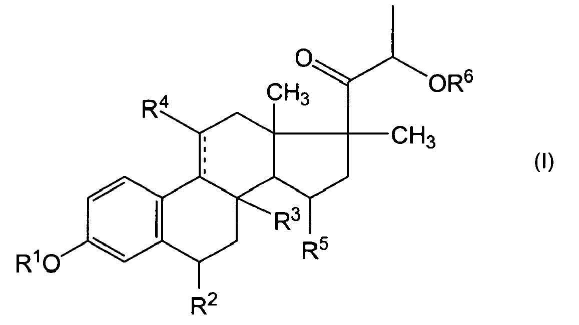 enthalten und Verfahren zur Kontrazeption durch Verabreichung dieser Darreichungsformen. Ferner betrifft die Erfindung Verbindungen der allgemeinen Formel (I) als Medikament und die Verwendung dieser Verbindungen zur Herstellung von Arzneimitteln zur Hormonersatz-Therapie.

Eine der am häufigsten angewendeten Methoden der Empfängnisverhütung (Kontrazeption) stellt die Verabreichung bzw. Applikation von Steroidhormonen wie Estrogenen in Kombination mit Gestagenen dar. Viele der heutzutage eingesetzten, kommerziell erhältlichen, hormonellen Kontrazeptiva weisen jedoch häufig Nebenwirkungen auf, welche dazu führen können, dass die Einnahme des Kontrazeptivums unterbrochen werden muss und somit keine effiziente Therapie bzw. Empfängnisverhütung mehr gewährleistet ist.

Eine Aufgabe der Erfindung besteht darin, Verbindungen zur Verfügung zu stellen, welche sich als pharmazeutische Wirkstoffe eignen und Vorteile gegenüber herkömmlichen pharmazeutischen Wirkstoffen aufweisen. Die pharmazeutischen Wirkstoffe sollten insbesondere zur Kontrazeption und/oder Hormonersatz-Therapie geeignet sein.

Diese Aufgabe wird durch den Gegenstand der Patentansprüche gelöst.

Es wurde überraschend gefunden, dass die Verbindungen der allgemeinen Formel (I) Affinität zum humanen Progesteronrezeptor aufweisen und daher insbesondere als pharmazeutische Wirkstoffe geeignet sind, beispielsweise zur Hormonersatz-Therapie oder zur Kontrazeption.

Ein Gegenstand der Erfindung betrifft eine pharmazeutische Zusammensetzung enthaltend
- wenigstens einen physiologisch verträglichen Hilfsstoff und
- wenigstens eine Verbindung der allgemeinen Formel (I) 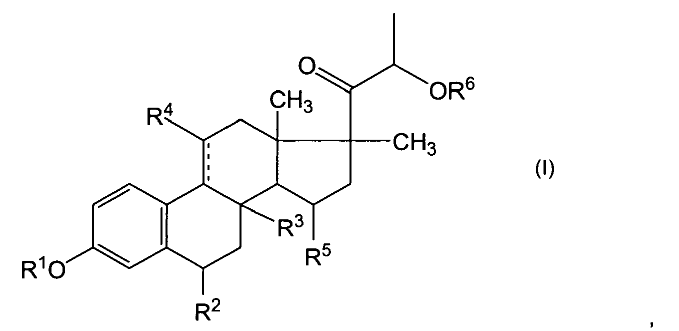
worin
R¹ und R⁶, jeweils unabhängig voneinander, für -H, -C₁-C₁₂-Kohlenwasserstoff oder -CO-C₁-C₁₂-Kohlenwasserstoff stehen;
R², R³, R⁴ und R⁵, jeweils unabhängig voneinander, für -H, -OH, -O-C₁-C₁₂-Kohlenwasserstoff oder -O-CO-C₁-C₁₂-Kohlenwasserstoff stehen;
"-----" für eine Einfachbindung oder eine Doppelbindung steht;
wobei der -C₁-C₁₂-Kohlenwasserstoff, jeweils unabhängig voneinander, aliphatisch und/oder aromatisch ist; und unsubstituiert ist oder mit einem oder mehreren Substituenten substituiert ist, welche unabhängig voneinander ausgewählt sind aus der Gruppe bestehend aus Halogen und -OH;

in Form eines Stereoisomers oder deren Mischung, jeweils in Form der freien Verbindungen oder physiologisch verträglichen Salze und/oder deren Solvate.

Sofern zum Zwecke der Beschreibung auf Verbindungen der allgemeinen Formel (I) verwiesen wird, sind die pharmazeutisch verträglichen Salze oder Solvate mit umfasst, auch wenn diese nicht jeweils ausdrücklich erwähnt werden.

Im Sinne der Beschreibung wird unter einem -C₁-C₁₂-Kohlenwasserstoff vorzugsweise ein Rest verstanden, welcher Kohlenstoff- und Wasserstoffatomen enthält und 1, 2, 3, 4, 5, 6, 7, 8, 9, 10, 11 oder 12 Kohlenstoffatome enthält.

Der Begriff "Kohlenwasserstoff" ist dem Fachmann bekannt und ist beispielsweise in G. P. Moss, Glossary of class names of organic compounds and reactive intermediates based on structure, Pure & Applied Chemistry 1995 (67) 1307-1375 definiert.

Bevorzugt ist der Kohlenwasserstoff aliphatisch oder aromatisch bzw. enthält sowohl einen aliphatischen als auch einen aromatischen Bestandteil. Bevorzugt ist der Kohlenwasserstoff gesättigt oder einfach oder mehrfach ungesättigt. Handelt es sich um einen aliphatischen Kohlenwasserstoff, so kann dieser acyclisch und/oder cyclisch (alicyclisch) sein. Handelt es sich um einen acyclischen Kohlenwasserstoff, so kann dieser linear oder verzweigt sein. Die aliphatischen Kohlenwasserszoffe, d.h. die linearen oder verzweigten acyclischen Kohlenwasserstoffe und die cyclischen (alicyclischen) Kohlenwasserstoffe, können jeweils gesättigt oder ein- oder mehrfach ungesättigt sein.

Somit ist im Sinne der Beschreibung unter einem aliphatischen Kohlenwasserstoff vorzugsweise ein acyclischer oder cyclischer (alicyclischer), gesättigter bzw. einfach oder mehrfach ungesättigter Kohlenwasserstoffrest zu verstehen, welcher nicht aromatisch ist. Zudem kann ein acyclischer aliphatischer Kohlenwasserstoff vorzugsweise unverzweigt (linear) oder verzweigt sein. Ist der aliphatische Kohlenwasserstoff ungesättigt, so kann er vorzugsweise wenigstens eine Doppelbindung und/oder wenigstens eine Dreifachbindung, vorzugsweise 1, 2 oder 3 Doppelbindungen und/oder Dreifachbindungen aufweisen. Geeignete gesättigte oder ungesättigte aliphatische -C₁-C₁₂-Kohlenwasserstoffreste sind beispielsweise Methyl, Ethyl, n-Propyl, iso-Propyl, n-Butyl, iso-Butyl, sec-Butyl, tert-Butyl, n-Pentyl, neo-Pentyl, n-Hexyl, Vinyl, Allyl, Ethinyl, Cyclopropyl, Cyclobutyl, Cyclopentyl, Cyclohexyl, Cycloheptyl, Cyclopentenyl und Cyclohexenyl.

Ein ungesättigter Kohlenwasserstoff kann eine oder mehrere konjugierte oder nicht-konjugierte C=C-Doppel- oder C≡C-Dreifachbindungen aufweisen (z.B. -CH=CH-CH=CH₂, -C≡C-C≡CH), bzw. gleichzeitig sowohl eine oder mehrere C=C-Doppelbindungen als auch eine oder mehrere C≡C-Dreifachbindungen (z.B. -CH=CH-CH₂-C≡CH), welche wiederum konjugiert oder nicht-konjugiert sein können.

Die Begriffe "aliphatisch" und "alicyclisch" sind dem Fachmann bekannt und sind beispielsweise in G. P. Moss, Glossary of class names of organic compounds and reactive intermediates based on structure, Pure & Applied Chemistry 1995 (67) 1307-1375 definiert.

Gesättigte, lineare oder verzweigte, acyclische aliphatische Kohlenwasserstoffe werden üblicherweise auch als "Alkyl" bezeichnet (z.B. Methyl, Ethyl, Propyl, Butyl). Entsprechend werden ungesättigte, lineare oder verzweigte, acyclische aliphatische Kohlenwasserstoffe, die wenigstens eine C=C-Doppelbindung aufweisen, üblicherweise auch als "Alkenyl" (z.B. Ethenyl, Propenyl, Vinyl, Allyl) und ungesättigte, lineare oder verzweigte, acyclische aliphatische Kohlenwasserstoffe, die wenigstens eine C≡C-Dreifachbindung aufweisen, werden üblicherweise auch als "Alkinyl" bezeichnet (z.B. Ethinyl).

Gesättigte, cyclische aliphatische Kohlenwasserstoffe werden üblicherweise auch als "Cycloalkyl" bezeichnet (z.B. Cyclopropyl, Cyclobutyl, Cyclopentyl, Cyclohexyl). Ungesättigte, cyclische, aliphatische Kohlenwasserstoffe, die wenigstens eine C=C-Doppelbindung aufweisen, werden üblicherweise als "Cycloalkenyl" bezeichnet (z.B. Cyclopentenyl, Cyclohexenyl).

Die Begriffe "Alkyl", "Alkenyl", "Alkinyl", "Cycloalkyl" und "Cycloalkenyl" sind dem Fachmann bekannt und sind beispielsweise in G. P. Moss, Glossary of class names of organic compounds and reactive intermediates based on structure, Pure & Applied Chemistry 1995 (67) 1307-1375 definiert.

Im Sinne der Beschreibung kann der aliphatische Kohlenwasserstoff vorzugsweise sowohl aus einem alicyclischen als auch aus einem acyclischen Bestandteil aufgebaut sein, welcher seinerseits linear oder verzweigt sein kann. In diesem Zusammenhang seien die Reste Cyclopentylmethyl, Cyclohexylethyl, Methylcyclopentyl und Ethylcyclohexyl beispielhaft erwähnt.

Aromatische Kohlenwasserstoffe sind dem Fachmann bekannt. Der aromatische Kohlenwasserstoff kann unkondensiert (nicht annelliert) oder kondensiert (annelliert) sein. Die Begriffe "aromatisch" und "annelliert" sind dem Fachmann bekannt und sind beispielsweise in P. Muller, Glossary of terms used in physical organic chemistry, Pure & Applied Chemistry 1994 (66) 1077-1184 definiert. Ein geeigneter aromatischer Kohlenwasserstoff, welcher unkondensiert (nicht annelliert) ist, ist beispielsweise Phenyl. Naphthyl ist ein Beispiel für einen kondensierten (annellierten) aromatischen Kohlenwasserstoff.

Im Sinne der Beschreibung versteht man unter einem aliphatischen und aromatischen Kohlenwasserstoff vorzugsweise einen Rest, welcher sowohl einen aliphatischen Bestandteil als auch einen aromatischen Bestandteil enthält, wobei die Begriffe aliphatischer und aromatischer Kohlenwasserstoff wie vorstehend beschrieben definiert sind. Geeignete Reste, die sowohl einen aliphatischen als auch einen aromatischen Kohlenwasserstoff enthalten, sind beispielsweise Benzyl, Methylphenyl, Dimethylphenyl, Mesityl, Phenethyl, Ethylphenyl, Phenylpropyl, Propylphenyl, Naphthylmethyl, Methylnaphthyl, Naphthylethyl und Ethylnaphthyl.

Im Sinne der Beschreibung ist der Kohlenwasserstoffrest der funktionellen Gruppe -CO-C₁-C₁₂-Kohlenwasserstoff vorzugsweise definiert wie vorstehend beschrieben. Vorzugsweise ist der Rest -CO-C₁-C₁₂-Kohlenwasserstoff ausgewählt aus der Gruppe bestehend aus Acetyl, Propionyl, Butyryl, Isobutyryl, Pentanoyl, 2-Methylbutanoyl, 3-Methylbutanoyl, Pivaloyl, Hexanoyl, Heptanoyl, Acryloyl, But-3-enoyl, Benzoyl, 2-Phenylacetyl und Naphthoyl.

Im Sinne der Beschreibung ist der Kohlenwasserstoffrest der funktionellen Gruppe -O-C₁-C₁₂-Kohlenwasserstoff vorzugsweise definiert wie vorstehend beschrieben. Vorzugsweise ist der Rest -O-C₁-C₁₂-Kohlenwasserstoff ausgewählt aus der Gruppe bestehend aus Methoxy, Ethoxy, Propoxy, iso-Propoxy, n-Butoxy, iso-Butoxy, sec-Butoxy, tert-Butoxy, n-Pentyloxy, neo-Pentyloxy, n-Hexyloxy, n-Heptyl, Vinyloxy, Allyloxy, Phenoxy, Benzyloxy, Phenethoxy, Naphthalen-1-yloxy und Naphthalen-2-yloxy.

Im Sinne der Beschreibung ist der Kohlenwasserstoffrest der funktionellen Gruppe -O-CO-C₁-C₁₂-Kohlenwasserstoff vorzugsweise definiert wie vorstehend beschrieben. Vorzugsweise ist der Rest -O-CO-C₁-C₁₂-Kohlenwasserstoff ausgewählt aus der Gruppe bestehend aus Acetoxy, Propionyloxy, Butyryloxy, Isobutyryloxy, Pentanoyloxy, 2-Methylbutanoyloxy, 3-Methylbutanoyloxy, Pivaloyloxy, Hexanoyloxy, Heptanoyloxy, Acryloyloxy, But-3-enoyloxy, Benzoyloxy, 2-Phenylacetoxy und Naphthoyloxy.

Unter dem Begriff "Halogen" wird im Sinne der Beschreibung vorzugsweise ein Rest ausgewählt aus der Gruppe bestehend aus -F, -Cl, -Br und -I verstanden.

Der Begriff "Stereoisomer" umfasst im Sinne der Beschreibung vorzugsweise Enantiomere und Diastereomere. Im Sinne der Beschreibung ist der Begriff "Epimer" vom Begriff "Diastereomer" vorzugsweise umfasst.

Im Sinne der Beschreibung steht der Begriff "Mischung aus Stereoisomeren" vorzugsweise für das Razemat oder Mischungen von Enantiomeren und/oder Diastereomeren in jedem beliebigen Verhältnis.

Die Begriffe "Enantiomer", "Diastereomer", "Epimer" und "Razemat" sind dem Fachmann bekannt und sind beispielsweise in G. P. Moss, Basic terminology of stereochemistry, Pure & Applied Chemistry 1996 (68) 2193-2222 definiert.

Unter dem Begriff "physiologisch verträgliches Salz" versteht man im Sinne der Beschreibung vorzugsweise Salze der erfindungsgemäßen Verbindungen, die - insbesondere bei Anwendung am Menschen und/oder Säugetier - toxikologisch unbedenklich sind.

In einer bevorzugten Ausführungsform enthält die erfindungsgemäße pharmazeutische Zusammensetzung wenigstens einen physiologisch verträglichen Hilfsstoff und wenigstens eine Verbindung der allgemeinen Formel (II) 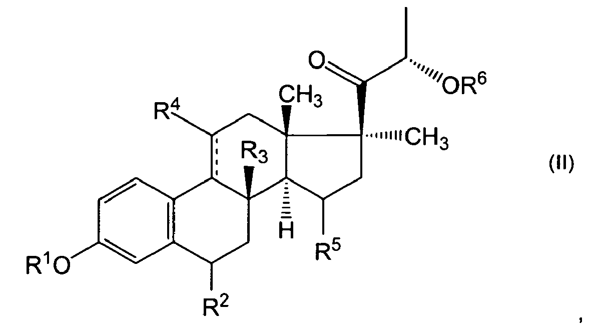 worin
R¹, R², R³, R⁴, R⁵, R⁶ und "-----" jeweils die vorstehend genannte Bedeutung haben; in Form der freien Verbindungen oder physiologisch verträglichen Salze und/oder deren Solvate.

In einer bevorzugten Ausführungsform sind bei den Verbindungen der allgemeinen Formel (I) oder (II) R², R³, R⁴ und R⁵, jeweils unabhängig voneinander, ausgewählt aus der Gruppe bestehend aus -H, -OH, Methoxy, Ethoxy, Propoxy, iso-Propoxy, n-Butoxy, iso-Butoxy, sec-Butoxy, tert-Butoxy, Phenoxy, Benzyloxy, Acetoxy, Propionyloxy, Butyryloxy, Isobutyryloxy, Pivaloyloxy, Hexanoyloxy, Heptanoyloxy und Benzoyloxy; bevorzugter -H oder -OH, besonders bevorzugt -H.

In einer anderen bevorzugten Ausführungsform sind bei den Verbindungen der allgemeinen Formel (I) oder (II) R¹ und R⁶, jeweils unabhängig voneinander, ausgewählt aus der Gruppe bestehend aus -H, Methyl, Ethyl, n-Propyl, iso-Propyl, n-Butyl, iso-Butyl, sec-Butyl, tert-Butyl, Phenyl, Benzyl, Acetyl, Propionyl, Butyryl, Isobutyryl, Pentanoyl, Pivaloyl, Hexanoyl, Heptanoyl und Benzoyl; bevorzugter -H, Methyl, Ethyl, n-Propyl, iso-Propyl, n-Butyl, tert-Butyl, Acetyl, n-Propionyl, n-Butyryl, Pivaloyl, n-Hexanoyl, n-Heptanoyl und Benzoyl; noch bevorzugter -H oder Acetyl; besonders bevorzugt -H.

In einer besonders bevorzugten Ausführungsform sind bei den Verbindungen der allgemeinen Formel (I) oder (II) R², R³, R⁴ und R⁵, jeweils unabhängig voneinander -H oder -OH, besonders bevorzugt -H; und R¹ und R⁶ jeweils -H oder Acetyl, besonders bevorzugt -H,
wobei "-----" vorzugsweise eine Einfach- oder Doppelbindung, besonders bevorzugt eine Doppelbindung ist.

In einer weiteren bevorzugten Ausführungsform enthält die erfindungsgemäße pharmazeutische Zusammensetzung eine Verbindung der allgemeinen Formel (III) 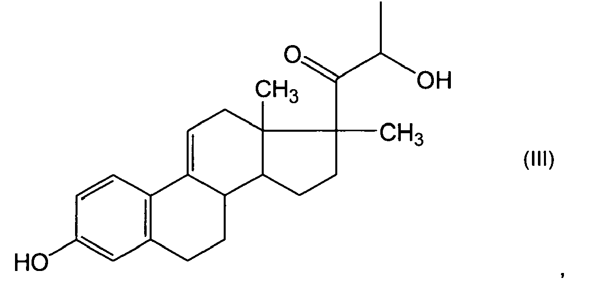 in Form eines Stereoisomers oder deren Mischung, d.h. 2-Hydroxy-1-(3-hydroxy-13,17-dimethyl-7,8,12,13,14,15,16,17-octahydro-6H-cyclopenta[a]phenanthren-17-yl)propan-1-on, jeweils in Form der freien Verbindungen oder physiologisch verträglichen Salze und/oder deren Solvate.

Weiterhin ist insbesondere bevorzugt, dass die erfindungsgemäße pharmazeutische Zusammensetzung die Verbindung der allgemeinen Formel (IV) 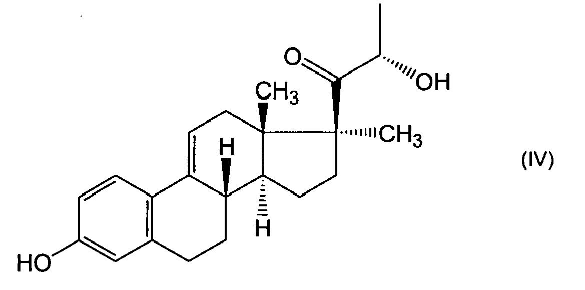 enthält, d.h. (S)-2-Hydroxy-1-((8S,13S,14S,17S)-3-hydroxy-13,17-dimethyl-7,8,12,13,14, 15,16,17-octahydro-6H-cyclopenta[a]phenanthren-17-yl)propan-1-on bzw. (2S,17S)-17-(2-Hydroxypropanoyl)-3-hydroxy-17-methylestra-1,3,5(10),9(11)-tetraen, in Form der freien Verbindung, deren physiologisch verträglichen Salze und/oder Solvate.

Falls die erfindungsgemäße pharmazeutische Zusammensetzung eine Mischung aus zwei Enantiomeren der allgemeinen Formel (I), (II), (III) oder (IV) enthält, beträgt der Enantiomerenüberschuss (e.e.) vorzugsweise mindestens 25%ee, bevorzugter mindestens 50%ee, noch bevorzugter mindestens 75%ee, am bevorzugtesten mindestens 90%ee und insbesondere mindestens 98%ee oder 100%ee.

Falls die erfindungsgemäße pharmazeutische Zusammensetzung zwei oder mehr Diastereomere der allgemeinen Formel (I), (II), (III) oder (IV) enthält, beträgt der Diastereomerenüberschuss (d.e.) vorzugsweise mindestens 25%de, bevorzugter mindestens 50%de, noch bevorzugter mindestens 75%de, am bevorzugtesten mindestens 90%de und insbesondere mindestens 98%de oder 100%ee.

Die Begriffe "Enantiomerenüberschuss" und "Diastereomerenüberschuss" sind dem Fachmann bekannt und sind beispielsweise in G. P. Moss, Basic terminology of stereochemistry, Pure & Applied Chemistry 1996 (68) 2193-2222 definiert.

Vorzugsweise weisen die Verbindungen der allgemeinen Formel (I), (II), (III) oder (IV) gegenüber dem Referenzstandard Progesteron eine relative Bindungsaffinität zum rekombinanten humanen Progestin-Rezeptor von mindestens 8,0, bevorzugter von mindestens 14, noch bevorzugter von mindestens 20, am bevorzugtesten von mindesten 26 und insbesondere von mindestens 32 auf. In einer besonders bevorzugten Ausführungsform beträgt die relative Bindungsaffinität zum Progestin-Rezeptor 35±4,0.

In einer weiteren bevorzugten Ausführungsform weisen die Verbindungen der allgemeinen Formel (I), (II), (III) oder (IV) gegenüber dem Referenzstandard Dexamethason eine relative Bindungsaffinität zum rekombinanten humanen Glucocorticoid-Rezeptor von höchstens 10, bevorzugter von höchstens 7,0, noch bevorzugter von höchstens 5,0, am bevorzugtesten von höchstens 3,0 und insbesondere von höchstens 1,0 auf. In einer besonders bevorzugten Ausführungsform beträgt die relative Bindungsaffinität zum Glucocorticoid-Rezeptor 0,70±0,10.

Desweiteren weisen die Verbindungen der allgemeinen Formel (I), (II), (III) oder (IV) gegenüber dem Referenzstandard Testosteron eine relative Bindungsaffinität zum rekombinanten humanen Androgen-Rezeptor von vorzugsweise höchstens 1,0, bevorzugter von höchstens 0,50, noch bevorzugter von höchstens 0,10, am bevorzugtesten von höchstens 0,070 und insbesondere von höchstens 0,050 auf. In einer besonders bevorzugten Ausführungsform beträgt die relative Bindungsaffinität zum Androgen-Rezeptor höchstens 0,030±0,0050.

Vorzugsweise weisen die Verbindungen der allgemeinen Formel (I), (II), (III) oder (IV) gegenüber dem Referenzstandard Aldosteron eine relative Bindungsaffinität zum rekombinanten humanen Mineralcorticoid-Rezeptor von höchstens 20, bevorzugter von höchstens 15, noch bevorzugter von höchstens 10, am bevorzugtesten von höchstens 7,0 und insbesondere von höchstens 4,0 auf. In einer besonders bevorzugten Ausführungsform beträgt die relative Bindungsaffinität zum Mineralcorticoid-Rezeptor 2,0±0,20.

In einer weiteren bevorzugten Ausführungsform weisen die Verbindungen der allgemeinen Formel (I), (II), (III) oder (IV) gegenüber dem Referenzstandard Estradiol eine relative Bindungsaffinität zum rekombinanten humanen Estrogen-Rezeptor von höchstens 5,0, bevorzugter von höchstens 1,0, noch bevorzugter von höchstens 0,75, am bevorzugtesten von höchstens 0,50 und insbesondere von höchstens 0,25 auf. In einer besonders bevorzugten Ausführungsform beträgt die relative Bindungsaffinität zum Estrogen-Rezeptor 0,18±0,010.

Bestimmungsmethoden zur Affinität an die vorstehend genannten Rezeptoren sind dem Fachmann bekannt. In diesem Zusammenhang kann beispielsweise verwiesen werden auf EP-A 808 845; I. Lacroix et al., Bioorganic & Medicinal Chemistry 1999 (7) 2329-2341; und D. Philibert et al., Gynecol Endocrinol 1999, 13, 316-26.

Die Selektivität der Verbindungen der allgemeinen Formel (I), (II), (III) oder (IV) für den Progestin-Rezeptor gegenüber dem Glucocorticoid-Rezeptor beträgt vorzugsweise mindestens 10, bevorzugter mindestens 20, noch bevorzugter mindestens 30, am bevorzugtesten mindestens 40 und insbesondere mindestens 50. Die Selektivität berechnet sich aus dem Quotienten aus relativer Bindungsaffinität am Progestin-Rezeptor und relativer Bindungsaffinität am Glucocorticoid-Rezeptor.

In einer bevorzugten Ausführungsform beträgt die Selektivität der Verbindungen der allgemeinen Formel (I), (II), (III) oder (IV) für den Progestin-Rezeptor gegenüber dem Androgen-Rezeptor vorzugsweise mindestens 100, bevorzugter mindestens 500, noch bevorzugter mindestens 700, am bevorzugtesten mindestens 900 und insbesondere mindestens 1.100. Die Selektivität berechnet sich aus dem Quotienten aus relativer Bindungsaffinität am Progestin-Rezeptor und relativer Bindungsaffinität am Androgen-Rezeptor.

In einer weiteren bevorzugten Ausführungsform beträgt die Selektivität der Verbindungen der allgemeinen Formel (I), (II), (III) oder (IV) für den Progestin-Rezeptor gegenüber dem Mineralcorticoid-Rezeptor vorzugsweise mindestens 5,0, bevorzugter mindestens 8,0, noch bevorzugter mindestens 11, am bevorzugtesten mindestens 14 und insbesondere mindestens 17. Die Selektivität berechnet sich aus dem Quotienten aus relativer Bindungsaffinität am Progestin-Rezeptor und relativer Bindungsaffinität am Mineralcorticoid-Rezeptor.

Vorzugsweise beträgt die Selektivität der Verbindungen der allgemeinen Formel (I), (II), (III) oder (IV) für den Progestin-Rezeptor gegenüber dem Estrogen-Rezeptor mindestens 40, bevorzugter mindestens 80, noch bevorzugter mindestens 120, am bevorzugtesten mindestens 160 und insbesondere mindestens 190. Die Selektivität berechnet sich aus dem Quotienten aus relativer Bindungsaffinität am Progestin-Rezeptor und relativer Bindungsaffinität am Estrogen-Rezeptor.

Es wurde überraschend gefunden, dass die Verbindungen der allgemeinen Formel (I), (II), (III) und (IV), insbesondere Verbindungen der allgemeinen Formel (III) und (IV), eine hohe Selektivität für den Progestin-Rezeptor aufweisen, d.h. sie binden mit hoher Affinität an den Progestin-Rezeptor, jedoch allenfalls mit geringer Affinität an andere Steroid-Rezeptoren, insbesondere Estrogen-Rezeptoren. Dies ist überraschend, da aufgrund der Estrogen-ähnlichen Struktur der erfindungsgemäßen Verbindungen (aromatischer Ring A wie in den drei natürlichen Estrogenen Estradiol, Estriol und Estron) eine hohe Selektivität für den Estrogen-Rezeptor zu erwarten gewesen wäre, was jedoch nicht der Fall ist. Somit handelt es sich bei den erfindungsgemäßen Verbindungen gewissermaßen strukturell um Estogen-Derivate, welche jedoch funktionell gestagene Wirkung haben.

Die Menge der wenigstens einen Verbindung der allgemeinen Formel (I), (II), (III) oder (IV) beträgt vorzugsweise mindestens 100 µg, bevorzugter mindestens 200 µg, noch bevorzugter mindestens 300 µg, am bevorzugtesten mindestens 400 µg und insbesondere mindestens 500 µg, bezogen auf das Gesamtgewicht der erfindungsgemäßen pharmazeutischen Zusammensetzung. In einer bevorzugten Ausführungsform liegt die Menge der wenigstens einen Verbindung der allgemeinen Formel (I), (II), (III) oder (IV) in der erfindungsgemäßen pharmazeutischen Zusammensetzung im Bereich von 500 µg bis 3.000 µg, bevorzugter von 510 bis 2.500 µg, noch bevorzugter von 525 bis 2.000 µg, am bevorzugtesten von 550 bis 1.500 µg und insbesondere von 600 bis 900 µg.

In einer anderen bevorzugten Ausführungsform entspricht die Menge der wenigstens einen Verbindung der allgemeinen Formel (I), (II), (III) oder (IV) in der erfindungsgemäßen pharmazeutischen Zusammensetzung einer Äquivalentdosis von Trimegeston von mindestens 100 µg, bevorzugter mindestens 200 µg, noch bevorzugter mindestens 300 µg, am bevorzugtesten mindestens 400 µg und insbesondere mindestens 500 µg. In einer bevorzugten Ausführungsform entspricht die Menge der wenigstens einen Verbindung der allgemeinen Formel (I), (II), (III) oder (IV) in der erfindungsgemäßen pharmazeutischen Zusammensetzung einer Äquivalentdosis von Trimegeston im Bereich von 500 µg bis 3.000 µg, bevorzugter von 510 bis 2.500 µg, noch bevorzugter von 525 bis 2.000 µg, am bevorzugtesten von 550 bis 1.500 µg und insbesondere von 600 bis 900 µg.

Die Äquivalentdosis der wenigstens einen Verbindung der allgemeinen Formel (I), (II), (III) oder (IV) im Vergleich zu Trimegeston wird dabei so gewählt, dass die gestagene Aktivität derjenigen entspricht, welche die Verabreichung von Trimegeston in der angegebenen Menge hervorrufen würde. Geeignete Verfahren zur Bestimmung der Äquivalentdosis sind dem Fachmann bekannt.

Vorzugsweise enthält die pharmazeutische Zusammensetzung die wenigstens eine Verbindung der allgemeinen Formel (I), (II), (III) oder (IV) in einer Menge von 0,0010 bis 99,999 Gew.-%, bevorzugter 0,10 bis 99,9 Gew.-%, noch bevorzugter 0,50 bis 75 Gew.-%, am bevorzugtesten 1,0 bis 50 Gew.-% und insbesondere 2,0 bis 25 Gew.-%, jeweils bezogen auf das Gesamtgewicht der pharmazeutischen Zusammensetzung.

Neben wenigstens einer Verbindung der allgemeinen Formel (I), (II), (III) oder (IV), ggf. jeweils in Form eines Stereoisomers oder deren Mischung, jeweils in Form der freien Verbindungen oder physiologisch verträglichen Salze und/oder deren Solvate, enthält die erfindungsgemäße pharmazeutische Zusammensetzung zusätzlich einen oder mehrere Hilfsstoffe, die vorzugsweise ausgewählt werden aus der Gruppe bestehend aus Salzbildnern, Puffern, Emulgatoren, Einbettungsmittel, Verdickungsmitteln, Penetrationspromotoren, Filmbildnern, Bindemitteln, Gleitmitteln, oberflächenaktiven Stoffen, Weichmachern, Zerfallsbeschleunigern, Lösungsmitteln, Befeuchtungsmitteln, Gelbildnern, Konservierungsmitteln, Stabilisatoren (Reduktionsmitteln, Antioxidantien), Formtrennmitteln, Füllmitteln, Schmiermittel, Chelatbildnern, Aromazusätzen, Duftstoffen und Farbstoffen.

Geeignete Puffer, die für die pharmazeutische Zusammensetzung verwendet werden können, sind dem Fachmann bekannt. So kann als Puffer beispielsweise ein Phosphat-, Acetat-oder Carbonatpuffer eingesetzt werden.

Emulgatoren werden vorzugsweise in solchen Mengen beigegeben, dass sie ein gleichmäßiges Vermischen der Komponenten der erfindungsgemäßen, pharmazeutischen Zusammensetzung ermöglichen. Übliche Emulgatoren umfassen vorzugsweise anionische, kationische und/oder nichtionische oberflächenaktive Substanzen. Beispiele für derartige Emulgatoren umfassen vorzugsweise Natriumstearat, Natriumlauryl-5-sulfat, Natriumacetylsulfat, Polyoxyethylenstearat, Polyoxyethylensorbitan-monostearat, Sorbitan, Propylenglycolmonostearat und/oder ethoxyliertes Lanolin. Der Emulgator wird vorzugsweise in einer Menge von 0,1 bis 10 Gew.-%, bezogen auf das Gesamtgewicht der erfindungsgemäßen pharmazeutischen Zusammensetzung eingesetzt.

Beispiele für Einbettungsmittel sind Carnaubawachs, Montanglycolwachs, Stearinpalmitinsäure, Glyceroltrioleat und Cetylstearylalkohol.

Verdickungsmittel, die vorzugsweise in der erfindungsgemäßen Zusammensetzung enthalten sein können, umfassen beispielsweise Candelilla, Carnauba- und mikrokristalline Wachse, Carbomer und Polyethylen-Verdicker. Das Verdickungsmittel wird vorzugsweise in einer Menge von 0,50 bis 2,0 Gew.-%, bezogen auf das Gesamtgewicht der erfindungsgemäßen pharmazeutischen Zusammensetzung eingesetzt.

Geeignete Penetrationspromotoren im Sinne der Beschreibung umfassen vorzugsweise Penetrationspromotoren, die ausgewählt sind aus der Gruppe umfassend Säureamide und Amine. Besonders bevorzugt wird als Penetrationspromotor Harnstoff eingesetzt. Der Penetrationspromotor wird vorzugsweise in einer Menge von 0,50 bis 10 Gew.-%, bezogen auf das Gesamtgewicht der erfindungsgemäßen pharmazeutischen Zusammensetzung eingesetzt.

Beispiele für Filmbildner sind Schellack, Methylcellulose, Hydroxypropylmethylcellulose (HPMC), Hydroxypropylcellulose, Hydroxyethylcellulose, Ethylcellulose, Polyacrylate und Polymethacrylate.

Geeignete Bindemittel sind beispielsweise Hydroxypropylcellulose, Stärke, Celluloseether, Polyvinylpyrrolidon (Povidon), Hydroxypropylmethylcellulose, Gelatine und Zucker (z.B. Saccharose, Glucosesirup). Die Bindemittel können einen Gewichtsanteil von bevorzugt 0,50 bis 5,0 Gew.-% bezogen auf das Gesamtgewicht der erfindungsgemäßen pharmazeutischen Zusammensetzung ausmachen.

Geeignete Gleitmittel sind beispielsweise Talk, langkettige Fettsäuren wie Stearinsäure und Palmitinsäure, deren Salze wie Magnesiumstearat und Calciumstearat, Polyethylenglykol und hydrierte pflanzliche Öle. Die Gleitmittel können einen Gewichtsanteil von bevorzugt 0,25 bis 3,0 Gew.-% bezogen auf das Gesamtgewicht der erfindungsgemäßen pharmazeutischen Zusammensetzung ausmachen.

Ein geeigneter Fließverbesserer ist z.B. kolloidales Siliziumdioxid. Die Fließverbesserer können einen Gewichtsanteil von bevorzugt 0,10 bis 3,0 Gew.-% bezogen auf das Gesamtgewicht der pharmazeutischen Zusammensetzung ausmachen.

Weiterhin können auch oberflächenaktive Stoffe (Tenside), die nichtionische, kationische, anionische und/oder ampholytische Eigenschaften besitzen, enthalten sein. Bevorzugt werden nichtionische oberflächenaktive Stoffe für die pharmazeutische Zusammensetzung verwendet, die vorzugsweise Sorbitanester, Polyoxyethylensorbitanester, Glycerinester, Polyoxyethylenglycerinether, Polyglycerinfettsäureester, Glycerinfettsäureester, Polyoxyethylenglycerinfettsäureester, Polyoxyethylen-verzweigte Alkylether, Polyoxyethylenalkylether, Polyoxyethylen-hydrogenierte Castorölfettsäureester, und/oder Polyoxyethylenalkylarylether umfassen.

Anionische oberflächenaktive Stoffe umfassen vorzugsweise Salze, wie Diethanolaminsalz und Triethanolaminsalz, höherer Fettsäuren, Ethercarbonsäure-alkaline Salze und N-Acylaminosäurensalze. Der oberflächenaktive Stoff wird vorzugsweise in einer Menge von 0,10 bis 10 Gew.-%, bezogen auf das Gesamtgewicht der erfindungsgemäßen pharmazeutischen Zusammensetzung eingesetzt.

Weichmacher können ebenfalls in der erfindungsgemäßen, pharmazeutischen Zusammensetzung enthalten sein. Übliche Weichmacher sind vorzugsweise ausgewählt aus der Gruppe umfassend Öle und Wachse, Silikonöle, Triglyceridester, Acetoglyceridester, ethoxylierte Glyceride, Alkylester, Alkenylester, Fettsäuren, Fettalkohole, Fettalkoholester, Lanolin und dessen Derivate, mehrfach hydrierte Alkohole und deren Ether, mehrfach hydrierte Alkoholester, Wachsester, Bienenwachs-Derivate, pflanzliche Wachse, Phospholipide, Sterole und Amide. Die Weichmacher werden vorzugsweise in einer Menge von 1,0 bis 25 Gew.-%, bezogen auf das Gesamtgewicht der erfindungsgemäßen pharmazeutischen Zusammensetzung eingesetzt.

Zerfallsbeschleuniger (Tablettensprengmittel) werden einer pharmazeutischen Zusammensetzung zugesetzt, um den Zerfall einer aus der Zusammensetzung hergestellten Darreichungsform zu begünstigen. Geeignete Sprengmittel sind beispielsweise modifizierte oder unmodifizierte Stärke, Tonmineralien, quervernetztes Polyvinylpyrrolidon, modifizierte oder unmodifizierte Cellulose, Gummi oder Algine. Die Tablettensprengmittel können einen Gewichtsanteil von bevorzugt 5,0 bis 50 Gew.-%, bevorzugter 5,0 bis 15 Gew.-% ausmachen bezogen auf das Gesamtgewicht der erfindungsgemäßen pharmazeutischen Zusammensetzung.

Lösungsmittel können in der erfindungsgemäßen pharmazeutischen Zusammensetzung enthalten sein, wie beispielsweise Wasser, Ethanol, Mischungen aus Wasser und Ethanol, Propylenglykol oder Glycerol. Bevorzugt ist die pharmazeutische Zusammensetzung jedoch frei von Lösungsmitteln, d.h. sie weist eine (Rest-)Feuchte von weniger als 10 Gew.-%, bevorzugter weniger als 5,0 Gew.-%, noch bevorzugter weniger als 2,0 Gew.-%, am bevorzugtesten weniger als 1,0 Gew.-% und insbesondere weniger als 0,50 Gew.-% auf bezogen auf das Gesamtgewicht der erfindungsgemäßen pharmazeutischen Zusammensetzung.

Ein Beispiel für ein Befeuchtungsmittel ist Glycerin.

Für die erfindungsgemäßen Zusammensetzungen geeignete Gelbildner umfassen vorzugsweise natürliche oder synthetische Polymere. Natürliche Polymere sind vorzugsweise ausgewählt aus der Gruppe umfassend Agar-Agar, Alginsäure, Alginat, Carbomer, Carrageenan, Dextrine, Gelatine, Guargummi, Gummi arabicum, Keratin, Pektin, Schellack, (ggf. modifizierte) Stärke, Traganth, Xanthangummi und deren Derivate. Bevorzugte synthetische Polymere, die als Geliermittel für die erfindungsgemäße Zusammensetzung eingesetzt werden können sind ausgewählt aus der Gruppe umfassend Acrylsäurepolymere, Carbomer, Polyacrylamide und Alkylenoxidpolymere. Die Gelbildner werden vorzugsweise in einer Menge von 0,10 bis 5,0 Gew.-%, bezogen auf das Gesamtgewicht der erfindungsgemäßen pharmazeutischen Zusammensetzung eingesetzt.

Konservierungsmittel können auch in der erfindungsgemäßen pharmazeutischen Zusammensetzung enthalten sein. Beispiele für Konservierungsmittel sind Alkohole (z.B. Ethanol, Phenylethylalkohol, Benzylalkohol), Säuren (z.B. Sorbinsäure oder Benzoesäure) und Phenolderivate (z.B. Phenol, Chlorkresol). Die Konservierungsmittel werden vorzugsweise in einer Menge von 0,0010 bis 15 Gew.-%, bezogen auf das Gesamtgewicht der erfindungsgemäßen pharmazeutischen Zusammensetzung verwendet.

Als Stabilisatoren können Antioxidantien und/oder Reduktionsmittel eingesetzt werden. Geeignete Reduktionsmittel sind beispielsweise Sulfite, Mercaptocarbonsäuren, Mercaptoamine, Mercaptoamide, Hydroxide, Alkohole, Di-thio-Verbindungen, Lithiumchlorid, 2-Mercaptoethansulfonsäure, Dithionit, Ameisensäure und Oxalsäure. Die Reduktionsmittel werden vorzugsweisein einer Menge von 0,0010 bis 2,0 Gew.-%, bezogen auf das Gesamtgewicht der erfindungsgemäßen pharmazeutischen Zusammensetzung verwendet.

Geeignete Antioxidantien sind beispielsweise Ascorbinsäure, Butylhydroxyanisol, Butylhydroxytoluol, Calcium-Di-Natrium-EDTA, Lecithin, Milchsäure, Tocopherol und Zitronensäure. Das Antioxidans wird vorzugsweise in einer Menge von 0,0010 bis 2,0 Gew.-%, bezogen auf das Gesamtgewicht der erfindungsgemäßen pharmazeutischen Zusammensetzung verwendet.

Geeignete Füllmittel sind beispielsweise Laktose, Mannit, Sorbit, Cellulose, mikrokristalline Cellulose, Xylit, Dextrose, Fructose, Stärke und Sucrose und Mischungen daraus. Die Füllmittel können einen Gewichtsanteil von bevorzugt 70 bis 95 Gew.-% bezogen auf das Gesamtgewicht der pharmazeutischen Zusammensetzung ausmachen.

Beispiele für Schmiermittel sind Stearinsäure, Magnesiumstearat, Calciumstearat und Zinkstearat.

Beispiele für Chelatbildner sind Zitronensäure, Phenylalanin, Natriumcalciumedetat und Dinatriumedetat (EDTA-Na₂).

Die pharmazeutische Zusammensetzung kann auch Duftstoffe bzw. Aromazusätze enthalten. Geeignete Duftstoffe bzw. Aromazusätze sind beispielsweise Citronellal, Citronellol, Eukalyptol, Geraniol, Limonen, Myrcenol, Pinen, Cedren, Eugenol und Vanillin. Der Duftstoff bzw. Aromazusatz wird vorzugsweise in einer Menge von 0,0010 bis 2,0 Gew.-% bezogen auf das Gesamtgewicht der erfindungsgemäßen pharmazeutischen Zusammensetzung eingesetzt.

Bevorzugt verwendete Farbstoffe für die pharmazeutische Zusammensetzung umfassen:
i) anorganische und organische Pigmente, wie beispielsweise Titanoxid, Zirkonoxid, Ceroxid, Zinkoxid, Eisenoxid, Preußisch-Blau, Kohleschwärze, Calciumlake und Aluminiumlake.
ii) fettlösliche Farbstoffe, wie beispielsweise Sudanrot, DC Red 17, DC Green 6, Beta-Caroten, Sojaöl, Sudanbraun, DC Yellow 11, DC Violet 2, DC Orange 5 und Quinoline Yellow.
iii) wasserlösliche Farbstoffe, wie beispielsweise Eisensulfate, Methylenblau und natürliche Farbstoffe.

Die Farbstoffe oder Farbstoffmischungen werden vorzugsweise in einer Menge von 0,0010 bis 2,0 Gew.-%, bezogen auf das Gesamtgewicht der erfindungsgemäßen pharmazeutischen Zusammensetzung eingesetzt.

In einer bevorzugten Ausführungsform enthält die erfindungsgemäße pharmazeutische Zusammensetzung neben wenigstens einer Verbindung der allgemeinen Formel (I), (II), (III) oder (IV) folgende Hilfsstoffe in folgenden bevorzugten Mengen (Prozentangaben sind auf das Gesamtgewicht der erfindungsgemäßen pharmazeutischen Zusammensetzung bezogen):

| Inhaltsstoff | bevorzugt [Gew.-%] | bevorzugter [Gew.-%] | insbesondere [Gew.-%] |
|---|---|---|---|
| HPMC | 1,0 bis 7,5 | 2,5 bis 5,0 | 3,0 bis 5,0 |
| Titandioxid | 0,1 bis 2,0 | 0,5 bis 1,5 | 0,7 bis 1,2 |
| Stärke | 10 bis 60 | 20 bis 40 | 25 bis 35 |
| Laktose | 25 bis 80 | 40 bis 70 | 50 bis 65 |
| Stearinsäure | 0,1 bis 2,5 | 0,2 bis 1,5 | 0,3 bis 1,0 |
| Talkum | 0,1 bis 5,0 | 0,5 bis 2,5 | 0,9 bis 1,5 |

Die Herstellung der erfindungsgemäßen pharmazeutischen Zusammensetzung erfolgt mittels der üblichen, aus dem Stande der Technik bekannten Mitteln, Vorrichtungen, Methoden und Verfahren, wie sie beispielsweise in *"*Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind.

In einer bevorzugten Ausführungsform enthält die pharmazeutische Zusammensetzung neben wenigstens einem physiologisch verträglichen Hilfsstoff und wenigstens einer Verbindung der allgemeinen Formel (I), (II), (III) oder (IV) zusätzlich wenigstens ein Gestagen, das vorzugsweise ausgewählt ist aus der Gruppe bestehend aus Allylestrenol, Chlormadinon, Cyproteron, Danazol, Demegeston, Desogestrel, Dienogest, Drospirenon, Dydrogesteron, Ethisteron, Etynodiol, Gestoden, Gestonoron, Hydroxyprogesteron, Levonorgestrel, Lynestrenol, Medroxyprogesteron, Medrogeston, Megestrol, Methylestrenolon, Methylnortestosteron, Nomegestrol, Norethisteron, Norethynodrel, Norgestrel, Norgestimat, Progesteron, Promegeston, Tibolon, Trimegeston, 1 β-Hydroxytrimegeston und 6β-Hydroxytrimegeston.

Bevorzugte pharmazeutisch verträgliche Ester der vorstehend aufgeführten Gestagene sind Acetate (z.B. Chlormadinonacetat, Medroxyprogesteronacetat, Megestrolacetat, Norethisteronacetat), Capronate (z.B. Hydroxyprogesteroncapronat) und Enanthate (z.B. Norethisteronenanthat).

In einer bevorzugten Ausführungsform enthält die pharmazeutische Zusammensetzung zusätzlich wenigstens ein Estrogen, welches vorzugsweise ausgewählt ist aus der Gruppe bestehend aus Chlorotrianisen, Dienestrol, Diethylstilbestrol, Estradiol (17β-Estradiol), Estriol, Estron, Ethinylestradiol, Estradiolbenzoat, Hexestrol, Mestranol, Methallenestril, Methylestrenol, Promestrien und konjugierten Estrogenen bzw. deren pharmazeutisch verträglichen Estern, wie beispielsweise Valerate. Als zusätzliche Estrogen-Komponente können insbesondere Ethinylestradiol bzw. eine Kombination aus Ethinylestradiol und Estradiol (17β-Estradiol) verwendet werden.

In einer bevorzugten Ausführungsform enthält die pharmazeutische Zusammensetzung eine Kombination aus wenigstens einem physiologisch verträglichen Hilfsstoff, wenigstens einer Verbindung der allgemeinen Formel (I), (II), (III) oder (IV) und wenigstens ein zusätzliches der vorstehend aufgeführten Gestagene und/oder wenigstens eines der vorstehend aufgeführten Estrogene. Besonders bevorzugt enthält die pharmazeutische Zusammensetzung eine Kombination aus wenigstens einem physiologisch verträglichen Hilfsstoff, wenigstens einer Verbindung der allgemeinen Formel (I), (II), (III) oder (IV) und Ethinylestradiol oder eine Kombination aus wenigstens einem physiologisch verträglichen Hilfsstoff, wenigstens einer Verbindung der allgemeinen Formel (I), (II), (III) oder (IV) und einer Kombination aus Ethinylestradiol und Estradiol (17β-Estradiol).

Die pharmazeutische Zusammensetzung kann flüssig (z.B. Lösung, Dispersion, Suspension, Emulsion), pastös, halbfest oder fest (z.B. Pulver, Granulat) sein. Vorzugsweise ist sie fest.

Vorzugsweise enthält die erfindungsgemäße pharmazeutische Zusammensetzung wenigstens ein eisenhaltiges Präparat, Folsäure und/oder Folinsäure.

Beispiele für eisenhaltige Präparate sind Eisen(II)-Präparate, wie z.B. Eisen(II)sulfat, Eisen(II)carbonat, Eisen(II)chlorid, Eisen(II)tartrat, Eisen(II)gluconat, Eisen(II)aspartat, Eisen(II)glycinsulfat, Eisen(II)fumarat, Eisen(II)ascorbat, Eisen(II)iodat, Eisen(II)succinat und Ammoniumeisen(II)sulfat; und Eisen(III)-Präparate, wie z.B. Eisen(III)-Natriumcitrat, Eisen(III)oxid-Sachharose-Komplex, Natriumferedetat, Eisen(III)hydroxid, Dextriferron, Eisen(III)citrat, Chondroitinsulfat-Eisen(III)-Komplex, Eisen(III)-Acetyltransferrin, Eisen(III)-Proteinsuccinylat und Kalium-Eisen(III)-Phosphat-Citrat-Komplex.

Die Folsäure bzw. deren Derivat kann in der erfindungsgemäßen pharmazeutischen Zusammensetzung vorzugsweise in freier Form oder als Salz, beispielsweise als Calciumfolat, vorliegen.

Ein weiterer Gegenstand der Erfindung betrifft eine pharmazeutische Darreichungsform umfassend die vorstehend beschriebene pharmazeutische Zusammensetzung.

Die erfindungsgemäße Darreichungsform ist vorzugsweise zur einmal, zweimal oder dreimal täglichen, besonders bevorzugt zur einmal oder zweimal täglichen Verabreichung konfektioniert. In einer insbesondere bevorzugten Ausführungsform ist die erfindungsgemäße Darreichungsform zur oralen Verabreichung konfektioniert. Besonders bevorzugt ist eine Darreichungsform, vorzugsweise zur oralen Verabreichung, enthaltend die vorstehend beschriebene pharmazeutische Zusammensetzung, welche in Form von Tageseinheiten zur Verfügung gestellt wird.

Die erfindungsgemäße pharmazeutische Darreichungsform kann vorzugsweise als flüssige, halbfeste oder feste Darreichungsform, beispielsweise in Form von Suspensionen, Salben, Cremes, Lotionen, Gelen, Emulsionen, Tabletten, Kapseln, Dragees, Pulvern, Suppositorien, Pflastern, Vaginalringen, Vaginalstäbchen, Implantaten, Intrauterinpessaren, Hormonspiralen, Spritzen oder Depot-Ampullen vorliegen. Die erfindungsgemäße Darreichungsform liegt vorzugsweise als Tablette, Filmtabelle, Dragee, Kapsel, Pelletformulierung, Suppositorium, Transdermalpflaster oder Vaginalring vor. Geeignete Ausführungsformen sind dem Fachmann grundsätzlich bekannt.

Sofern die pharmazeutische Darreichungsform fest ist, kann diese auch in vorzugsweise multipartikulärer Form, beispielsweise in Form von Mikrotabletten, Mikrokapseln, Mikropellets, Aufbaupellets, Granulaten, Extrudaten, Sphäroiden, Perlen oder Pellets vorliegen, ggf. in Kapseln abgefüllt oder zu (Film-)Tabletten verpresst sein, wobei auch Trockenkompaktierungen möglich sind.

Sofern die pharmazeutische Darreichungsform flüssig oder pastös ist, kann diese beispielsweise als Flüssigkeit, Schaum, Creme, Gel, Paste, Balsam, Spray, Salbe, Lotion, Spülung ("Conditioner"), Tonikum, Tinktur, Milch, Mus, Pulver zum Auflösen, Emulsion (Öl-in-Wasser, Wasser-in-Öl), Serum, Öl, Shampoo, Suspension, wie Liposome oder Nanosome, oder als Dispersion vorliegen.

Die erfindungsgemäße Darreichungsform kann die wenigstens eine Verbindung der allgemeinen Formel (I), (II), (III) oder (IV) sofort (*immediate release*) oder kontrolliert (*controlled release*) freisetzen. Erfolgt die Freisetzung kontrolliert, so kann sie beispielsweise zeitlich verzögert (*delayed release*), hinhaltend (*sustained release, prolonged release*) oder gepulst (*pulsed release, repeat action release*) erfolgen.

Sofern die erfindungsgemäße Darreichungsform Hilfsstoffe beinhaltet, so entsprechen diese den vorstehend aufgeführten Hilfsstoffen, welche auch für die Formulierung der erfindungsgemäßen pharmazeutischen Zusammensetzung eingesetzt werden können.

In einer bevorzugten Ausführungsform enthält die erfindungsgemäße Darreichungsform vorzugsweise in Form von Tageseinheiten, vorzugsweise zur oralen Verabreichung, die wenigstens eine Verbindung der allgemeinen Formel (I), (II), (III) oder (IV) in einer Menge von 0,0010 bis 99,999 Gew.-%, bevorzugter 0,10 bis 99,9 Gew.-%, noch bevorzugter 0,50 bis 75 Gew.-%, am bevorzugtesten 1,0 bis 50 Gew.-% und insbesondere 2,0 bis 25 Gew.-%, jeweils bezogen auf das Gesamtgewicht der Darreichungsform.

Die in der erfindungsgemäßen Darreichungsform enthaltene Dosis der wenigstens einen Verbindung der allgemeinen Formel (I), (II), (III) oder (IV) beträgt bevorzugt mindestens 100 µg, bevorzugter mindestens 200 µg, noch bevorzugter mindestens 300 µg, am bevorzugtesten mindestens 400 µg und insbesondere mindestens 500 µg. In einer bevorzugten Ausführungsform liegt die Dosis der wenigstens einen Verbindung der allgemeinen Formel (I), (II), (III) oder (IV) in der erfindungsgemäßen Darreichungsform vorzugsweise im Bereich von 500 µg bis 3.000 µg, bevorzugter von 510 bis 2.500 µg, noch bevorzugter von 525 bis 2.000 µg, am bevorzugtesten von 550 bis 1.500 µg und insbesondere von 600 bis 900 µg. In einer bevorzugten Ausführungsform liegt die wenigstens eine Verbindung der allgemeinen Formel (I), (II), (III) oder (IV) in einer solchen Menge vor, dass ihre Dosis der Äquivalentdosis von Trimegeston im vorstehend definierten Bereich entspricht.

Liegt die wenigstens eine Verbindung der allgemeinen Formel (I), (II), (III) oder (IV) in Kombination mit wenigstens einem Gestagen in der erfindungsgemäßen Darreichungsform vor, so entspricht die Dosis des Estrogens in der Darreichungsform vorzugsweise einer äquivalenten Dosis von 100 bis 5.000 µg, bevorzugter 250 bis 4.000 µg, noch bevorzugter 500 bis 3.500 µg, am bevorzugtesten 750 bis 3.000 µg und insbesondere 1.000 bis 2.500 µg Chlormadinonacetat. Sind mehrere Gestagene enthalten, so entspricht deren Gesamtdosis vorzugsweise den vorstehend genannten Äquivalentdosen.

Die Äquivalentdosis zu Chlormadinonacetat kann durch eine äquivalente Menge jedes geeigneten Gestagens verwirklicht werden, wobei die Menge dabei so gewählt wird, dass die gestagene Aktivität derjenigen entspricht, welche die Verabreichung von Chlormadinonacetat in der angegebenen Menge hervorrufen würde. Es ist auch möglich dass zwei oder mehrere unterschiedliche Gestagene in einer Menge eingesetzt werden, die insgesamt der angegebenen Äquivalentdosis entspricht. Geeignete Verfahren zur Bestimmung der Äquivalentdosis sind dem Fachmann bekannt.

Im Falle, dass die erfindungsgemäße Darreichungsform zusätzlich ein Gestagen enthält, kann das relative Mengenverhältnis der Trimegeston-Äquivalentdosis der wenigstens einen Verbindung der allgemeinen Formel (I), (II), (III) oder (IV) zur Chlormadinonacetat-Äquivalentdosis des zusätzlichen Gestagens vorzugsweise im Bereich von 30:1,0 bis 1,0:50, bevorzugter im Bereich von 20:1,0 bis 1,0:40, noch bevorzugter im Bereich von 10:1,0 bis 1,0:30, am bevorzugtesten im Bereich von 5,0:1,0 bis 1,0:20 und insbesondere im Bereich von 2,0:1,0 bis 1,0:10 liegen.

Liegt die wenigstens eine Verbindung der allgemeinen Formel (I), (II), (III) oder (IV) in Kombination mit wenigstens einem Estrogen vor, vorzugsweise mit Ethinylestradiol, so entspricht die Dosis des Estrogens in der Darreichungsform vorzugsweise einer äquivalenten Dosis von 5,0 bis 55 µg, bevorzugter 10 bis 50 µg, noch bevorzugter 15 bis 48 µg, am bevorzug-testen 20 bis 45 µg und insbesondere 22 bis 40 µg Ethinylestradiol. Sind mehrere Estrogene enthalten, so entspricht deren Gesamtdosis vorzugsweise den vorstehend genannten Äquivalentdosen.

Die Äquivalentdosis zu Ethinylestradiol kann durch eine äquivalente Menge jedes geeigneten Estrogens verwirklicht werden, wobei die Menge dabei so gewählt wird, dass die estrogene Aktivität, vorzugsweise die Ovulationsinhibition, derjenigen entspricht, welche die Verabreichung von Ethinylestradiol in der angegebenen Menge hervorrufen würde. Es ist auch möglich, dass zwei oder mehrere unterschiedliche Estrogene, beispielsweise Ethinylestradiol in Kombination mit Estradiol, in einer Menge eingesetzt werden, die insgesamt der angegebenen Äquivalentdosis entspricht. Geeignete Verfahren zur Bestimmung der Äquivalentdosis sind dem Fachmann bekannt.

Im Falle, dass die erfindungsgemäße Darreichungsform zusätzlich ein Estrogen enthält, kann das relative Mengenverhältnis der Trimegeston-Äquivalentdosis der wenigstens einen Verbindung der allgemeinen Formel (I), (II), (III) oder (IV) zur Ethinylestradiol-Äquivalentdosis des zusätzlichen Estrogens vorzugsweise im Bereich von 60:1,0 bis 1,5:1,0, bevorzugter im Bereich von 40:1,0 bis 1,4:1,0, noch bevorzugter im Bereich von 20:1,0 bis 1,3:1,0, am bevorzugtesten im Bereich von 10,0:1,0 bis 1,3:1,0 und insbesondere im Bereich von 5,0:1,0 bis 1,2:1,0 liegen.

Besonders bevorzugte Ausführungsformen für Kombinationen einer täglichen Dosierung X von wenigstens einer Verbindung der allgemeinen Formel (I), (II), (III) oder (IV) mit der täglichen Dosierungen Y von Ethinylestradiol, welche in der erfindungsgemäßen pharmazeutischen Darreichungsform enthalten sein können, sind in nachfolgender Tabelle zusammengefasst:

| Verbindung der allgemeinen Formel (I), (II), (III) oder (IV) | Ethinylestradiol |
|---|---|
| 500 ≤ X ≤ 3.000 µg | 10 µg ≤ Y ≤ 50 µg |
| 510 ≤ X ≤ 2.500 µg | 12 µg ≤ Y ≤ 48 µg |
| 525 ≤ X ≤ 2.000 µg | 15 µg ≤ Y ≤ 45 µg |
| 550 ≤ X ≤ 1.500 µg | 18 µg ≤ Y ≤ 42 µg |
| 600 ≤ X ≤ 900 µg | 20 µg ≤ Y ≤ 40 µg |

In einer bevorzugten Ausführungsform liegt die wenigstens eine Verbindung der allgemeinen Formel (I), (II), (III) oder (IV) in einer solchen Menge vor, dass ihre Dosis der Äquivalentdosis X von Trimegeston im vorstehend definierten Bereich entspricht.

Besonders bevorzugte Ausführungsformen für Kombinationen der täglichen Dosierung X von wenigstens einer Verbindung der allgemeinen Formel (I), (II), (III) oder (IV) mit der täglichen Dosierungen Y von Ethinylestradiol und der täglichen Dosierung Z von Estradiol (17β-Estradiol), welche in der erfindungsgemäßen Darreichungsform enthalten sein können, sind in nachfolgender Tabelle zusammengefasst:

| Verbindung der allgemeinen Formel (I), (II), (III) oder (IV) | Ethinylestradiol | Estradiol |
|---|---|---|
| 500 ≤ X ≤ 3.000 µg | 1,0 µg ≤ Y ≤ 10 µg | 1.000 µg ≤ Z ≤ 10.000 µg |
| 510 ≤ X ≤ 2.500 µg | 2,0 µg ≤ Y ≤ 10 µg | 1.100 µg ≤ Z ≤ 9.000 µg |
| 525 ≤ X ≤ 2.000 µg | 3,0 µg ≤ Y ≤ 9,5 µg | 1.200 µg ≤ Z ≤ 8.000 µg |
| 550 ≤ X ≤ 1.500 µg | 4,0 µg ≤ Y ≤ 9,5 µg | 1.300 µg ≤ Z ≤ 7.000 µg |
| 600 ≤ X ≤ 900 µg | 5,0 µg ≤ Y ≤ 9,0 µg | 1.400 µg ≤ Z ≤ 6.000 µg |

In einer bevorzugten Ausführungsform liegt die wenigstens eine Verbindung der allgemeinen Formel (I), (II), (III) oder (IV) in einer solchen Menge vor, dass ihre Dosis der Äquivalentdosis X von Trimegeston im vorstehend definierten Bereich entspricht.

Ein weiterer Gegenstand der Erfindung betrifft eine kosmetische Zusammensetzung, welche wenigstens eine Verbindung der allgemeinen Formel (I), (II), (III) oder (IV) enthält. Bevorzugt dient die kosmetische Zusammensetzung der Pflege der Haut und/oder der Haare, vorzugsweise durch topische Anwendung.

Als kosmetische Hilfsstoffe sind vorzugsweise die üblichen Hilfsstoffe solcher Zusammensetzungen geeignet. In diesem Zusammenhang kann beispielsweise vollumfänglich verwiesen werden auf H.P. Fiedler, Lexikon der Hilfsstoffe für Pharmazie, Kosmetik und angrenzende technische Gebiete, Editio Cantor Aulendorff, 2002. Vorzugsweise können die vorstehend aufgeführten Hilfsstoffe zum Einsatz kommen, welche auch in der erfindungsgemäßen pharmazeutischen Zusammensetzungen enthalten sein können. Diese Hilfsstoffe sind physiologisch verträglich und die Mengen der jeweiligen Komponenten sind vorzugsweise so gewählt, dass die erfindungsgemäße, kosmetische Zusammensetzung konform mit der EU Kosmetikrichtlinie 76/768/EEC bzw. der EU-Richtlinie 95/17/EC ist.

Die Auswahl der Hilfsstoffe sowie deren einzusetzende Mengen hängt davon ab, ob die erfindungsgemäße pharmazeutische bzw. kosmetische Zusammensetzung oral, topisch, subkutan, parenteral, intradermal, vaginal oder lokal appliziert bzw. verabreicht werden soll,
wobei eine orale, vaginale, subkutane, oder transdermalen Anwendung besonders bevorzugt ist. Oral oder perkutan anwendbare Zubereitungsformen können die Verbindung der allgemeinen Formel (I) auch verzögert freisetzen.

Ein weiterer Gegenstand der Erfindung betrifft Verbindungen der allgemeinen Formel (I) und (II) wie vorstehend definiert, mit der Maßgabe, dass wenn R¹, R², R³, R⁴ und R⁵ jeweils -H sind, R⁶ nicht -CO-CH₃ (Acetyl) ist (vgl. I. Lacroix et al., Bioorg. Med. Chem. 1999, 7, 2329-41, Verbindung 11), sowie die Verbindungen der allgemeinen Formel (III) und (IV) wie vorstehend definiert.

Die vorstehenden Ausführungen und Definitionen hinsichtlich der Verbindungen der allgemeinen Formeln (I), (II), (III) und (IV) der erfindungsgemäßen pharmazeutischen Zusammensetzung gelten analog auch für die entsprechenden erfindungsgemäßen Verbindungen.

Ein weiterer Gegenstand der Erfindung betrifft eine Verbindung der allgemeinen Formel (I), (II), (III) oder (IV) wie vorstehend definiert als Medikament.

Ein weiterer Gegenstand der Erfindung betrifft eine Verbindung der allgemeinen Formel (I), (II), (III) oder (IV) wie vorstehend definiert, bzw. deren Verwendung, ggf. in Kombination mit wenigstens einem Estrogen und/ oder einem weiteren Gestagen, zur Behandlung und/oder Vorbeugung von wenigstens einer der Beschwerden bzw. Erkrankungen ausgewählt aus der Gruppe bestehend aus Rosazea; Psoriasis; Blutungsstörungen; Dysmenorrhoe (Regelbeschwerden); menstruationszyklusabhängigen Erkrankungen, wie Endometriose, polyzystischem, ovariellen Syndrom (PCOS), Uterus myomatosus, funktionellen Zysten, menstruationszyklusabhängigen Stimmungsschwankungen, prämenstrueller dysphorischer Störung (PMDD), prämenstruellem Syndrom (PMS) und Kopfschmerzen/Migräne; menstruationszyklusbeeinflussten Erkrankungen, wie Epilepsie, Multiple Sklerose, Diabetes mellitus, Depression, Schizophrenie, Asthma und Morbus Parkinson; und androgeninduzierten Störungen, wie Seborrhoe, Akne, androgenetischer Alopezie und Hirsutismus.

Ein weiterer Gegenstand der Erfindung betrifft die erfindungsgemäße Verbindung der allgemeinen Formel (I), (II), (III) oder (IV), bzw. deren Verwendung, ggf. in Kombination mit wenigstens einem Estrogen und/oder wenigstens einem Gestagen, zur Hormonersatz-Therapie (HRT).

Ein weiterer Gegenstand der Erfindung betrifft die Verwendung von wenigstens einer Verbindung der allgemeinen Formel (I), (II), (III) oder (IV), ggf. in Kombination mit wenigstens einem Estrogen und/ oder einem weiteren Gestagen, zur Herstellung einer vorstehend beschriebenen pharmazeutischen Zusammensetzung oder Darreichungsform, zur Behandlung und/oder Vorbeugung von wenigstens einer der Beschwerden bzw. Erkrankungen ausgewählt aus der Gruppe bestehend aus Rosazea; Psoriasis; Blutungsstörungen; Dysmenorrhoe (Regelbeschwerden); menstruationszyklusabhängigen Erkrankungen, wie Endometriose, polyzystischem, ovariellen Syndrom (PCOS), Uterus myomatosus, funktionellen Zysten, menstruationszyklusabhängigen Stimmungsschwankungen, prämenstrueller dysphorischer Störung (PMDD), prämenstruellem Syndrom (PMS) und Kopfschmerzen/Migräne; menstruationszyklusbeeinflussten Erkrankungen, wie Epilepsie, Multiple Sklerose, Diabetes mellitus, Depression, Schizophrenie, Asthma und Morbus Parkinson; und androgeninduzierten Störungen, wie Seborrhoe, Akne, androgenetischer Alopezie und Hirsutismus.

Ein weiterer Gegenstand der Erfindung betrifft die Verwendung einer erfindungsgemäßen Verbindung der allgemeinen Formel (I), (II), (III) oder (IV), ggf. in Kombination mit wenigstens einem Estrogen und/oder wenigstens einem Gestagen, zur Herstellung einer pharmazeutischen Zusammensetzung oder einer Darreichungsform zur Hormonersatz-Therapie (HRT),
wobei die pharmazeutische Zusammensetzung und die Darreichungsform wie vorstehend beschreiben definiert sind.

Ein weiterer Gegenstand der Erfindung betrifft ein Verfahren zur Kontrazeption umfassend die Verabreichung einer erfindungsgemäßen Darreichungsform wie vorstehend definiert an Frauen im gebärfähigen Alter an wenigstens 21 aufeinanderfolgenden Tagen, beginnend an Tag 1 des Menstruationszyklus'. Die Verabreichung der erfindungsgemäßen Darreichungsform erfolgt hierbei vorzugsweise oral.

In einer bevorzugten Ausführungsform des erfindungsgemäßen Verfahrens wird an zumindest einem, vorzugsweise an allen der wenigstens 21 aufeinander folgenden Tage wenigstens eine Verbindung der allgemeinen Formel (I), (II), (III) oder (IV) in Kombination mit zumindest einem Estrogen verabreicht, wobei das Estrogen vorzugsweise ausgewählt ist aus der Gruppe bestehend aus Chlorotrianisen, Dienestrol, Diethylstilbestrol, Estradiol (17β-Estradiol), Estriol, Estron, Ethinylestradiol, Estradiolbenzoat, Hexestrol, Mestranol, Methallenestril, Methylestrenol, Promestrien und konjugierten Estrogenen bzw. deren physiologisch verträglichen Estern, wie beispielsweise Valerate. Insbesondere bevorzugt umfasst die zusätzliche Estrogenkomponente Ethinylestradiol bzw. eine Kombination aus Ethinylestradiol und Estradiol (17β-Estradiol), wobei die Menge der Estrogenkomponente vorzugsweise einer äquivalenten Dosis von 5,0 bis 55 µg, bevorzugter 10 bis 50 µg, noch bevorzugter 15 bis 48 µg, am bevorzugtesten 20 bis 45 µg und insbesondere 22 bis 40 µg Ethinylestradiol entspricht. Werden mehrere Estrogene eingesetzt, so entspricht deren tägliche Gesamtdosierung vorzugsweise den vorstehend genannten Äquivalentdosen.

In einer besonders bevorzugten Ausführungsform des erfindungsgemäßen Verfahrens erfolgt an keinem Tag der zumindest 21 aufeinander folgenden Tage die Verabreichung eines Estrogens ohne die Verabreichung von wenigstens einer erfindungsgemäßen Verbindung der Formel (I), (II), (III) oder (IV).

In einer bevorzugten Ausführungsform des erfindungsgemäßen Verfahrens ist an jedem der wenigstens 21, bevorzugter wenigstens 22, noch bevorzugter wenigstens 23, am bevorzugtesten wenigstens 24 und insbesondere wenigstens 25 aufeinanderfolgenden Tage die tägliche Dosierung der wenigstens einen Verbindung der Formel (I), (II), (III) oder (IV) gleich (= einphasiges Schema), wobei die Verabreichung vorzugsweise jeweils in Kombination mit zumindest einem Estrogen erfolgt.

In einer anderen bevorzugten Ausführungsform des erfindungsgemäßen Verfahrens werden die wenigstens 21, bevorzugter wenigstens 22, noch bevorzugter wenigstens 23, am bevorzugtesten wenigstens 24 und insbesondere wenigstens 25 aufeinanderfolgenden Tage in zwei, drei oder mehr Gruppen von Tagen aufgeteilt, wobei an allen Tagen innerhalb einer Gruppe die tägliche Dosierung der wenigstens einen Verbindung der allgemeinen Formel (I), (II), (III) oder (IV) gleich ist, jedoch an aufeinander folgenden Tagen unterschiedlicher Gruppen die tägliche Dosierung der wenigstens eine Verbindung der allgemeinen Formel (I), (II), (III) oder (IV) unterschiedlich ist (= mehrphasiges Schema), und wobei die Verabreichung vorzugsweise jeweils in Kombination mit zumindest einem Estrogen erfolgt.

Bevorzugte Schemata sind in nachfolgender Tabelle aufgeführt, wobei die tägliche Dosis der wenigstens einen Verbindung der allgemeinen Formel (I), (II), (III) oder (IV) A1, A2 bzw. A3 und die tägliche Dosis des zumindest einen Estrogens B ist:

| | | Anzahl der Phasen | | | | | | | |
|---|---|---|---|---|---|---|---|---|---|
| | | 1 | 2 | 2 | 3 | 3 | 3 | 4 | 4 |
| Ausführungsform Nr. | | 1 | 2₁ | 2₂ | 3₁ | 3₂ | 3₃ | 4₁ | 4₂ |
| Dauergesamt [Tage von 28] | | 21-25 | 21-25 | 21-25 | 21-25 | 21-25 | 21-25 | 21-25 | 21-25 |
| 1 | Dauer [Tage] | 21-25 | 7-13 | 7-13 | 3-8 | 3-8 | 3-8 | 3-8 | 3-8 |
| | Dosis Verbindung Formel (I), (II), (III) oder (IV) | A1 | A2 | A1 | A2 | A2 | A1 | A2 | A2 |
| | Dosis Estrogen (Äquivalentdosis zu Ethinylestradiol) | B | B | B | B | B | B | B | B |
| 2 | Dauer [Tage] | | 12-18 | 12-18 | 4-15 | 4-15 | 4-15 | 4-15 | 4-15 |
| | Dosis Verbindung Formel (I), (II), (III) oder (IV) | | A1 | A2 | A2 | A1 | A2 | A1 | A2 |
| | Dosis Estrogen (Äquivalentdosis zu Ethinylestradiol) | | B | B | B | B | B | B | B |
| 3 | Dauer [Tage] | | | | 4-15 | 4-15 | 4-15 | 4-15 | 4-15 |
| | Dosis Verbindung Formel (I), (II), (III) oder (IV) | | | | A1 | A2 | A2 | A2 | A1 |
| | Dosis Estrogen (Äquivalentdosis zu Ethinylestradiol) | | | | B | B | B | B | B |
| 4 | Dauer [Tage] | | | | | | | 2-5 | 2-5 |
| | Dosis Verbindung Formel (I), (II), (III) oder (IV) | | | | | | | A3 | A3 |
| | Dosis Estrogen (Äquivalentdosis zu Ethinylestradiol) | | | | | | | B | B |

Die jeweiligen Wertebereiche der Dosierungen für die jeweiligen Kombinationen von A1, A2, A3 und B für jede einzelne dieser Ausführungsformen Nr. 1, 2₁, 2₂, 3₁, 3₂, 3₃, 4₁ und 4₂ sind nachfolgender Tabelle zu entnehmen, wobei die Dosierung B des zumindest einen Estrogens als Äquivalentdosis zu Ethinylestradiol angegeben ist:

| | bevorzugt | bevorzugter | noch bevorzugter | insbesondere |
|---|---|---|---|---|
| A1 | 510-990 µg | 525-975 µg | 550-950 µg | 550-750 µg |
| A2 | 40-990 µg | 40-750 µg | 120-750 µg | 260-500 µg |
| A3 | 0-990 µg | 0-750 µg | 0-500 µg | 260-500 µg |
| B | 5,0-55 µg | 10-50 µg | 20-45 µg | 25-40 µg |

In einer bevorzugten Ausführungsform liegt die Verbindung der allgemeinen Formel (I), (II), (III) oder (IV) in einer solchen Menge vor, dass ihre Dosis der Äquivalentdosis A1, A2 bzw. A3 von Trimegeston im vorstehend definierten Bereich entspricht.

In einer bevorzugten Ausführungsform des erfindungsgemäßen Verfahrens erfolgt die Verabreichung der wenigstens einen Verbindung der allgemeinen Formel (I), (II), (III) oder (IV) nicht an allen Tagen des vorzugsweise 28-tägigen Menstruationszyklus'. Vielmehr ist es bevorzugt, dass an den Tagen, welche sich an die wenigstens 21 aufeinander folgenden Tage anschließen,
- ein Placebo,
- ein eisenhaltiges pharmazeutisch verträgliches Präparat oder
- ein Folsäure-haltiges Präparat verabreicht wird;
- oder überhaupt keine Verabreichung erfolgt.

Auf diese Weise wird erreicht, dass der Menstruationszyklus durch die Entzugsblutung beendet wird, so dass ein neuer Menstruationszyklus beginnen kann. Bevorzugt ist der Menstruationszyklus 28-tägig.

Gemäß einer anderen bevorzugten Ausführungsform des erfindungsgemäßen Verfahrens ist es jedoch auch möglich, dass der Menstruationszyklus länger als 28-tägig ist. Dies kann erfindungsgemäß dadurch erreicht werden, dass die Absetzung der wenigstens einen Verbindung der allgemeinen Formel (I), (II), (III) oder (IV) (und ggf. zumindest eines Estrogens und/oder zumindest eines weiteren Gestagens) erst zu einem späteren Zeitpunkt erfolgt, so dass die Entzugsblutung auch erst zu einem späteren Zeitpunkt stattfindet und damit der Menstruationszyklus auch erst zu einem späteren Zeitpunkt endet. In dieser Ausführungsform erfolgt die Verabreichung der wenigstens einen Verbindung der allgemeinen Formel (I), (II), (III) oder (IV) bevorzugt an mehr als 28 aufeinander folgenden Tagen.

Bevorzugt erfolgt die (ununterbrochene) Verabreichung der wenigstens einen Verbindung der allgemeinen Formel (I), (II), (III) oder (IV) bei dieser Ausführungsform an wenigstens 42 oder 56, bevorzugter wenigstens 63, noch bevorzugter wenigstens 84, am bevorzugtesten wenigstens 105 oder 112 und insbesondere wenigstens 126 oder 140 aufeinander folgenden Tagen, so dass innerhalb dieses Zeitraums keine Auslösung der Entzugsblutung beabsichtigt ist. Der zusammenhängende Zeitraum, in dem eine tägliche Verabreichung der wenigstens einen Verbindung der allgemeinen Formel (I), (II), (III) oder (IV) erfolgt, kann erfindungsgemäß auch noch länger sein. So ist es grundsätzlich möglich, eine Verabreichung der wenigstens einen Verbindung der allgemeinen Formel (I), (II), (III) oder (IV) über ein Jahr oder mehrere Jahre hinweg an allen aufeinander folgenden Tagen vorzunehmen, ohne dass es zu einer Entzugsblutung kommt.

In einer bevorzugten Ausführungsform des erfindungsgemäßen Verfahrens wird zumindest an einem der wenigstens 21 aufeinander folgenden Tage die wenigstens eine Verbindung der allgemeinen Formel (I), (II), (III) oder (IV) in Kombination mit zumindest einem weiteren Gestagen verabreicht. Die bei einer derartigen Kombination eingesetzten Gestagene sowie deren entsprechende, jeweilige Dosierungen entsprechen jenen der vorstehend aufgeführten, erfindungsgemäßen pharmazeutischen Zusammensetzung.

Das erfindungsgemäße Verfahren wird während mindestens eines Menstruationszyklus' durchgeführt. Bevorzugt wird das erfindungsgemäße Verfahren während mehrerer, insbesondere während mindestens 6 aufeinander folgenden Menstruationszyklen durchgeführt.

Ein weiterer Aspekt der Erfindung betrifft ein Kit umfassend zumindest eine der vorstehend beschriebenen, erfindungsgemäßen Darreichungsformen. Bevorzugt ist das erfindungsgemäße Kit zur jeweils einmal täglichen Verabreichung der darin enthaltenen Darreichungsformen ausgelegt.

Das Kit ist bevorzugt so zusammengestellt, dass mit Hilfe der in dem Kit enthaltenen, erfindungsgemäßen Darreichungsformen das vorstehend beschriebene, erfindungsgemäße Verfahren zur Kontrazeption durchgeführt werden kann, ohne dass weitere, erfindungsgemäße Darreichungsformen, welche nicht in dem Kit enthalten sind, beschafft werden müssen. Bevorzugt enthält das Kit für einen Tag jeweils eine Darreichungsform, da die Verabreichung bevorzugt einmal täglich erfolgt.

Ist der Menstruationszyklus 28-tägig, so umfasst das erfindungsgemäße Kit bevorzugt wenigstens so viele erfindungsgemäße Darreichungsformen wie erforderlich sind, um wenigstens eine Verbindung der allgemeinen Formel (I), (II), (III) oder (IV) an wenigstens 21 aufeinander folgenden Tagen eines 28-tägigen Menstruationszyklus' zu verabreichen. Wird die wenigstens eine Verbindung der allgemeinen Formel (I), (II), (III) oder (IV) an weniger als 28 Tagen verabreicht, so kann das erfindungsgemäße Kit für die verbleibenden Tage bis zum Ablauf der 28 Tage des Menstruationszyklus' entweder überhaupt keine Darreichungsformen, eisenhaltige Präparate, Folsäure-haltige Präparate oder Placebos, vorzugsweise ein eisenhaltiges Präparat, enthalten. Dabei ist es erforderlich, dass zumindest eine der Darreichungsformen des erfindungsgemäßen Kits eine erfindungsgemäße Darreichungsform ist.

Ist der Menstruationszyklus verlängert, d.h. ist er mehr als 28-tägig, so ist in dem erfindungsgemäßen Kit die Anzahl der enthaltenden Darreichungsformen entsprechend vergrößert,
wobei wiederum wenigstens eine der enthaltenden Darreichungsformen eine vorstehend beschriebene erfindungsgemäße Darreichungsform ist.

In einer bevorzugten Ausführungsform umfasst das erfindungsgemäße Kit alle Darreichungsformen, welche zur Verabreichung von wenigstens einer Verbindung der allgemeinen Formel (I), (II), (III) oder (IV) für mindestens einen oder zwei, bevorzugter mindestens drei, noch bevorzugter mindestens vier, am bevorzugtesten mindestens fünf und insbesondere mindestens sechs Menstruationszyklen erforderlich sind.

In einer bevorzugten Ausführungsform ist das erfindungsgemäße Kit für eine ein- oder mehrphasige Verabreichung von wenigstens einer Verbindung der allgemeinen Formel (I), (II), (III) oder (IV) in Kombination mit einem Estrogen ausgelegt. Der Menstruationszyklus ist dabei bevorzugt 28-tägig. Bei den zwei-, drei und vierphasigen Schemata ist die tägliche Dosierung der wenigstens einen Verbindung der allgemeinen Formel (I), (II), (III) oder (IV) und des Estrogens an allen Tagen innerhalb einer Phase jeweils konstant und an zwei aufeinanderfolgenden Tagen verschiedener Phasen verschieden.

Bevorzugte Ausführungsformen Nr. 1, 2₁, 2₂, 3₁, 3₂, 3₃, 4₁ und 4₂ des erfindungsgemäßen Kits umfassen insgesamt 21-25 erfindungsgemäße Darreichungsformen, wobei diese je nach Anzahl der Phasen die wenigstens eine erfindungsgemäße Verbindung der allgemeinen Formel (I), (II), (III) oder (IV) in den Dosierungen A1, A2, A3 und zumindest ein Estrogen in der Dosierung B gemäß nachfolgender Tabelle enthalten:

| | | Anzahl der Phasen | | | | | | | |
|---|---|---|---|---|---|---|---|---|---|
| | | 1 | 2 | 2 | 3 | 3 | 3 | 4 | 4 |
| Ausführungsform Nr. | | 1 | 2₁ | 2₂ | 3₁ | 3₂ | 3₃ | 4₁ | 4₂ |
| Darreichungsformen insgesamt | | 21-25 | 21-25 | 21-25 | 21-25 | 21-25 | 21-25 | 21-25 | 21-25 |
| 1 | Anzahl der Dosiseinheiten | 21-25 | 7-13 | 7-13 | 3-8 | 3-8 | 3-8 | 3-8 | 3-8 |
| | Dosis Verbindung Formel (I), (II), (III) oder (IV) | A1 | A2 | A1 | A2 | A2 | A1 | A2 | A2 |
| | Dosis Estrogen (Äquivalentdosis zu Ethinylestradiol) | B | B | B | B | B | B | B | B |
| 2 | Anzahl der Dosiseinheiten | | 12-18 | 12-18 | 4-15 | 4-15 | 4-15 | 4-15 | 4-15 |
| | Dosis Verbindung Formel (I), (II), (III) oder (IV) | | A1 | A2 | A2 | A1 | A2 | A1 | A2 |
| | Dosis Estrogen (Äquivalentdosis zu Ethinylestradiol) | | B | B | B | B | B | B | B |
| 3 | Anzahl der Dosiseinheiten | | | | 4-15 | 4-15 | 4-15 | 4-15 | 4-15 |
| | Dosis Verbindung Formel (I), (II), (III) oder (IV) | | | | A1 | A2 | A2 | A2 | A1 |
| | Dosis Estrogen (Äquivalentdosis zu Ethinylestradiol) | | | | B | B | B | B | B |
| 4 | Anzahl der Dosiseinheiten | | | | | | | 2-5 | 2-5 |
| | Dosis Verbindung Formel (I), (II), (III) oder (IV) | | | | | | | A3 | A3 |
| | Dosis Estrogen (Äquivalentdosis zu Ethinylestradiol) | | | | | | | B | B |

Die jeweiligen Wertebereiche der Dosierungen für die jeweiligen Kombinationen von A1, A2, A3 und B für jede einzelne dieser Ausführungsformen Nr. 1, 2₁, 2₂, 3₁, 3₂, 3₃, 4₁ und 4₂ sind nachfolgender Tabelle zu entnehmen, wobei die Dosierung B des zumindest einen

Estrogens als Äquivalentdosis zu Ethinylestradiol angegeben ist:

| | bevorzugt | bevorzugter | noch bevorzugter | insbesondere |
|---|---|---|---|---|
| A1 | 510-990 µg | 525-975 µg | 550-950 µg | 550-750 µg |
| A2 | 40-990 µg | 40-750 µg | 120-750 µg | 260-500 µg |
| A3 | 0-990 µg | 0-750 µg | 0-500 µg | 260-500 µg |
| B | 5,0-55 µg | 10-50 µg | 20-45 µg | 25-40 µg |

In einer bevorzugten Ausführungsform liegt die wenigstens eine Verbindung der allgemeinen Formel (I), (II), (III) oder (IV) in einer solchen Menge vor, dass ihre Dosis der Äquivalentdosis A1, A2 bzw. A3 von Trimegeston im vorstehend definierten Bereich entspricht.

Bevorzugt wird die wenigstens eine Verbindung der allgemeinen Formel (I), (II), (III) oder (IV) in Kombination mit Ethinylestradiol oder in Kombination mit Ethinylestradiol und Estradiol (17β-Estradiol) eingesetzt.

Bei den zwei-, drei und vierphasigen Schemata ist die tägliche Dosierung der wenigstens einen Verbindung der allgemeinen Formel (I), (II), (III) oder (IV) und des Estrogens an allen Tagen innerhalb einer Phase jeweils konstant und an zwei aufeinanderfolgenden Tagen verschiedener Phasen verschieden.

Die folgenden Beispiele dienen zur näheren Erläuterung der Erfindung, sind jedoch nicht einschränkend auszulegen.

### (2S,17S)-17-(2-Hydroxypropanoyl)-3-hydroxy-17-methylestra-1,3,5(10),9(11)-tetraen

Die Titelverbindung kann durch Acetylierung von 1-β-Hydroxytrimegeston, Aromatisierung des so erhaltenen Produkts und anschließende alkalische Verseifung erhalten werden. Dazu kann die aromatisierte Zwischenstufe (vgl. I. Lacroix et al., Bioorg. Med. Chem. 1999, 7, 2329-2341, phenolische Verbindung 11) verseift werden:

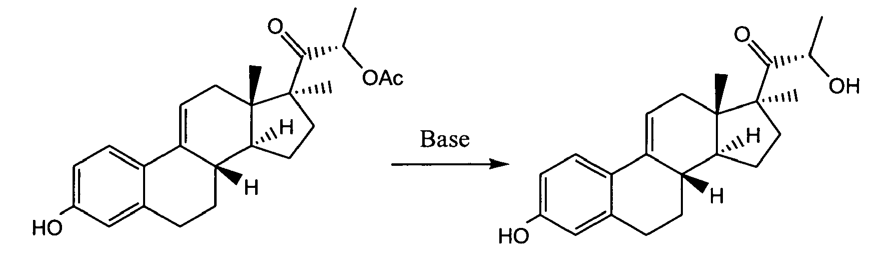

Geeignete Bedingungen zur alkalischen Esterverseifung sind dem Fachmann bekannt. In diesem Zusammenhang kann beispielsweise verweisen werden auf J. March, Advanced Organic Chemistry, 4th ed., John Wiley & Sons, Inc., 1992, pp 378-383.

### Relative Bindungsaffinität und Selektivität

Die relativen Bindungsaffinitäten von (2S,17S)-17-(2-Hydroxypropanoyl)-3-hydroxy-17-methylestra-1,3,5(10),9(11)-tetraen [Verbindung der allgemeinen Formel (IV)] an den rekombinanten humanen Progestin-Rezeptor, Mineralcorticoid-Rezeptor, Androgen-Rezeptor, Glucocorticoid-Rezeptor und Estrogen-Rezeptor wurden experimentell bestimmt (vgl. D. Philibert et al., Gynecol Endocrinol 1999, 13, 316-26).

Es wurde gefunden, dass (2S,17S)-17-(2-Hydroxypropanoyl)-3-hydroxy-17-methylestra-1,3,5(10),9(11)-tetraen bei hoher Selektivität eine deutliche Affinität an den Progestin-Rezeptor aufweist. Dies ist insbesondere hinsichtlich des Estrogen-Rezeptors überraschend, da aufgrund der strukturellen Ähnlichkeit zwischen Estrogenen und den erfindungsgemäßen Verbindungen eine höhere Affinität an den Estrogen-Rezeptor und damit eine geringere Selektivität für den Progestin-Rezeptor zu erwarten gewesen wäre.

## Patentansprüche

1. Pharmazeutische Zusammensetzung enthaltend wenigstens einen physiologisch verträglichen Hilfsstoff und wenigstens eine Verbindung der allgemeinen Formel (I) 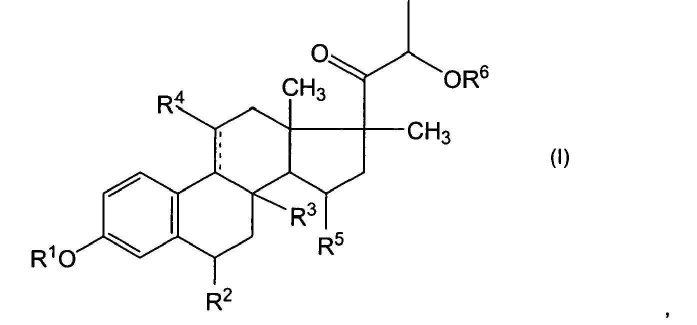 worin
R¹ und R⁶, jeweils unabhängig voneinander, für -H, -C₁-C₁₂-Kohlenwasserstoff oder -CO-C₁-C₁₂-Kohlenwasserstoff stehen;
R², R³, R⁴ und R⁵ jeweils unabhängig voneinander, für -H, -OH, -O-C₁-C₁₂-Kohlenwasserstoff oder -O-CO-C₁-C₁₂-Kohlenwasserstoff stehen;
"-----" für eine Einfachbindung oder eine Doppelbindung steht;
wobei der -C₁-C₁₂-Kohlenwasserstoff, jeweils unabhängig voneinander, aliphatisch und/oder aromatisch sein kann; und unsubstituiert ist oder mit einem oder mehreren Substituenten substituiert ist, welche unabhängig voneinander ausgewählt sind aus der Gruppe bestehend aus Halogen und -OH;
in Form eines Stereoisomers oder deren Mischung, jeweils in Form der freien Verbindungen oder physiologisch verträglichen Salze und/oder deren Solvate.

2. Zusammensetzung nach Anspruch 1, **dadurch gekennzeichnet, dass** R², R³, R⁴ und R⁵ jeweils für -H stehen.

3. Zusammensetzung nach Anspruch 1 oder 2, **dadurch gekennzeichnet, dass** R¹ und R⁶ für -H stehen.

4. Zusammensetzung nach einem der vorstehenden Ansprüche, **dadurch gekennzeichnet, dass** die Verbindung der allgemeinen Formel (I) gegenüber dem Referenzstandard Progesteron eine relative Bindungsaffinität zum Progestin-Rezeptor von mindestens 8,0 aufweist.

5. Zusammensetzung nach einem der vorstehenden Ansprüche, **dadurch gekennzeichnet, dass** der physiologisch verträgliche Hilfsstoff ausgewählt ist aus der Gruppe bestehend aus Salzbildnern, Puffern, Emulgatoren, Einbettungsmitteln, Verdickungsmitteln, Penetrationspromotoren, Filmbildnern, Bindemitteln, Gleitmitteln, oberflächenaktiven Stoffen, Weichmachern, Zerfallsbeschleunigern, Lösungsmitteln, Befeuchtungsmitteln, Gelbildnern, Konservierungsmitteln, Stabilisatoren, Formtrennmitteln, Füllmitteln, Schmiermitteln, Chelatbildnern, Aromazusätzen, Duftstoffen und Farbstoffen.

6. Zusammensetzung nach einem der vorstehenden Ansprüche, **dadurch gekennzeichnet, dass** sie zusätzlich wenigstens Estrogen enthält.

7. Zusammensetzung nach Anspruch 6, **dadurch gekennzeichnet, dass** das Estrogen Ethinylestradiol umfasst.

8. Pharmazeutische Darreichungsform umfassend eine pharmazeutische Zusammensetzung nach einem der vorstehenden Ansprüche.

9. Darreichungsform nach Anspruch 8, **dadurch gekennzeichnet, dass** sie zur ein- oder zweimal täglichen Verabreichung konfektioniert ist.

10. Darreichungsform nach Anspruch 8 oder 9, **dadurch gekennzeichnet, dass** sie zur oralen Verabreichung konfektioniert ist.

11. Zusammensetzung nach einem der Ansprüche 1 bis 7 oder Darreichungsform nach einem der Ansprüche 8 bis 10, **dadurch gekennzeichnet, dass** sie fest, halbfest oder flüssig ist.

12. Verbindung der allgemeinen Formel (I) definiert wie in einem der Ansprüche 1 bis 4, mit der Maßgabe, dass wenn R¹, R², R³, R⁴ und R⁵ jeweils -H sind, R⁶ nicht -CO-CH₃ ist.

13. Verbindung der allgemeinen Formel (1) definiert wie in einem der Ansprüche 1 bis 4 als Medikament.

14. Verwendung einer Verbindung nach Anspruch 12 zur Herstellung einer pharmazeutischen Zusammensetzung nach einem der Ansprüche 1 bis 7 oder einer Darreichungsform nach einem der Ansprüche 8 bis 10 zur Hormonersatztherapie.

15. Verfahren zur Kontrazeption umfassend die Verabreichung einer Darreichungsform nach einem der Ansprüche 8 bis 10 an Frauen im gebärfähigen Alter an wenigstens 21 aufeinander folgenden tagen, beginnend an Tag 1 des Menstruationszyklus'.
